# EUROPEAN PATENT APPLICATION

(11) **EP 4 339 955 A1**
(43) Date of publication of application: **20.03.2024**
(21) Application number: 22195620.4
(22) Date of filing: 14.09.2022
(51) Int. Cl.: G16B 20/20, G16B 40/20, G16B 15/20

(54) **RISK STRATIFICATION METHOD AND DEVICE**

(71) Applicant: Koninklijke Philips N.V., 5656 AG Eindhoven (NL)
(72) Inventor: CHAKRABARTI, Biswaroop, Eindhoven (NL)
(74) Representative: Philips Intellectual Property & Standards

(57) **Abstract**

Some embodiments are directed to risk stratification, e.g., of subjects on the basis of risk related to a disease with a genetic basis. Transcriptomic and/or genomic sequencing data is obtained for a biological sample, from which folding data is obtained. A machine-learning model applies to the folding data to obtain a risk score indicating a risk for in the subject.

## Description

### TECHNICAL FIELD

The presently disclosed subject matter relates to a method of risk stratification, a device for risk stratification, a computer readable medium.

### BACKGROUND

A genome is the genetic information of an organism. For humans, this comprises the chromosomal and mitochondrial DNA. Genome sequencing typically involves DNA extraction, purification, amplification, and sequencing into short reads which are later aligned to a reference sequence to obtain the final sequence. The set of all RNA transcripts, including coding and non-coding, in an individual or a population of cells is called the transcriptome.

The central dogma of molecular biology is often stated, simplified, as 'DNA makes RNA and RNA makes protein'. Genomic information causes effects through proteins. For example, from DNA to RNA to a peptide which folds into the final protein structure. The last step of protein formation involves folding of the nascent peptide chain-also referred to as the primary structure-firstly through hydrogen bond formations-also referred to as the secondary structure-and finally into a complex three-dimensional conformation-also referred to as the tertiary structure. In case of multi-subunit proteins, the subunits can arrange to form the final structure, knowns as the quaternary structure.

There is evidence of improper protein folding influencing the pathogenesis of several diseases, including malignancies. A particular example can be found in the paper by M. D. Scott and J. Frydman, "Aberrant Protein Folding as the Molecular Basis of Cancer". The paper explains that tumorigenesis often arises from mutations that interfere with the appropriate function of tumor-suppressor proteins. In particular, alterations in the folding of tumor-suppressor proteins or oncogenes may lead to carcinogenesis. For example, misfolded tumor suppressor proteins VHL and NF2 may lead to cancer as a result of a loss-of-function phenotype.

Though genetic and genomic information are now becoming part of clinical management, analysis of the effect of any change in the genome or transcriptome is not a part of clinical practice.

### SUMMARY

It would be advantageous to have an improved way to use transcriptomic and/or genomic sequencing data to better stratify subjects according to risk. Though genetic information is effected in the cell through proteins, application of genetic information in clinical practice is restricted. In an embodiment, in-silico protein structure prediction is used in clinical use for early identification of conditions where the pathogenesis involves aberrant protein folding.

For example, in a method of risk stratification, transcriptomic and/or genomic sequencing data may be obtained for at least one biological sample of a subject, say, a blood sample. Whereas one could try to use the sequencing data directly to estimate risk scores, in an embodiment, corresponding folding data is obtained first. The folding data indicates an expected 3d-structure of the protein if it were synthesized in the subject. Interestingly, the folding data therefore indicative of the correct or incorrect folding of the protein. The particular folding may be used to establish a risk score. For example, a machine-learning model may be applied to the folding data to obtain a first risk score. This model works in particular for diseases in which incorrect folding plays a role, e.g., diseases with a genetic basis in the subject, in particular cancer, e.g., a particular form of cancer, e.g., breast cancer, lung cancer, colorectal cancer, prostate cancer, amongst others.

Many genetic mutations do not lead to a folded protein that is so aberrant that it leads to disease, e.g., cancer. From the sequencing data itself this is hard to see, as folding is a computationally hard task, and it is unlikely that a machine learning algorithm trained to generate risk score will learn all the folding intricacies from a training signal comprising solely sequencing data as inputs and, e.g., outcome data as output. However, a folding function may be incorporated in the device, so that folding data can be provided as an input to a risk scoring algorithm. For example, an in-silico protein folding prediction algorithm may be applied to the sequencing data. This alleviates the machine learning model from the burden of having to learn folding intricacies. The desired output, e.g., risk score, is learned better, if folding data is provided as an input. Typically, one or more computer hardware processor will be configured to perform the protein folding, and machine learning training and/or applying.

Such a machine learning model may be obtained, by obtaining observed disease risk in a subject associated with folding data and training and/or retraining the machine-learning model to generate a risk score indicating the disease risk from at least folding data. The machine learning model receives folding data as an input. Additional inputs are possible, for example, other signals correlated with the disease for which a risk score is desired. In an embodiment, the machine learning model is configured to receive the sequencing data itself as an additional input. In an embodiment, the machine learning model is configured not to receive the sequencing data itself as an additional input.

The machine learning model may be configured to receive as input features computed from the folding data. For example, a device may identify structural features of the protein from the folding data. The machine-learning model may receive at least the identified structural features as input.

A risk stratifying device is an electronic device. The risk stratifying device may comprise a computer. A method of risk stratifying described herein may be applied in a wide range of practical applications. Such practical applications include clinical settings. For example, in a hospital or other care environment, risk stratifying may be used to prioritize treatment, order subjects, and so on.

An aspect is a method of risk stratifying. An embodiment of the method may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium. Another aspect of the presently disclosed subject matter is a method of making the computer program available for downloading.

### BRIEF DESCRIPTION OF DRAWINGS

Further details, aspects, and embodiments will be described, by way of example only, with reference to the drawings. Elements in the figures are illustrated for simplicity and clarity and have not necessarily been drawn to scale. In the figures, elements which correspond to elements already described may have the same reference numerals. In the drawings,
Figure 1 schematically shows an example of an embodiment of a device for risk stratification,
Figure 2a schematically shows an example of an embodiment of a device for risk stratification,
Figure 2b schematically shows an example of an embodiment of a device for risk stratification,
Figure 2c schematically shows an example of an embodiment of a device for risk stratification,
Figure 3 schematically shows an example of an embodiment of risk stratification,
Figure 4 schematically shows an example of an embodiment of risk stratification,
Figure 5 schematically shows an example of an embodiment of method of risk stratification,
Figure 6a schematically shows a computer readable medium having a writable part comprising a computer program according to an embodiment,
Figure 6b schematically shows a representation of a processor system according to an embodiment.

### Reference signs list

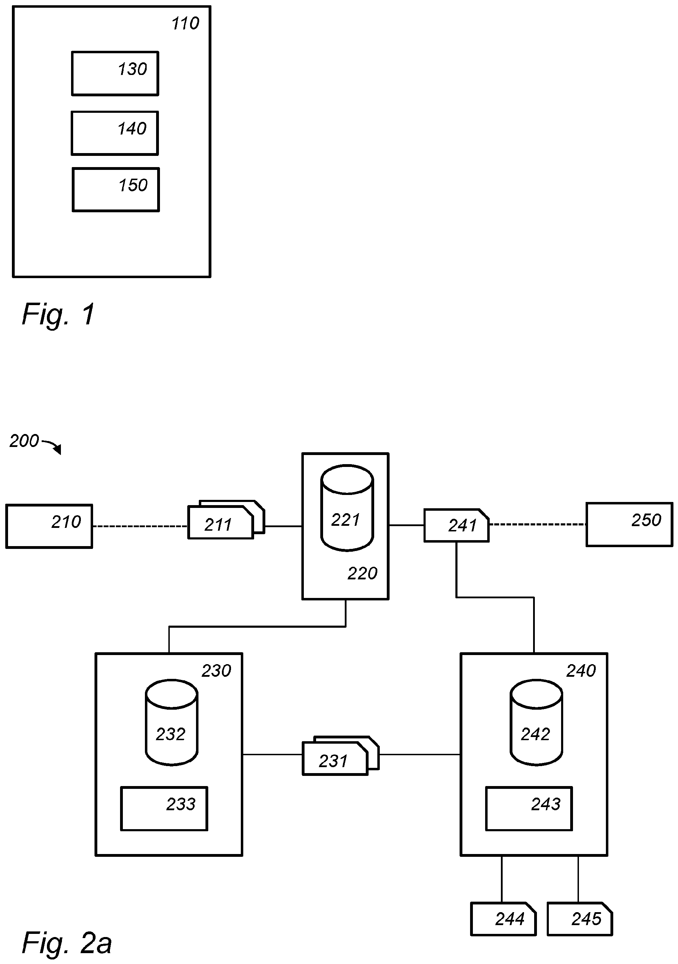

The following list of references and abbreviations corresponds to figures 1, 2a, 2b,2c, 6a and 6b and is provided for facilitating the interpretation of the drawings and shall not be construed as limiting the claims.
- 110: a device for risk stratification
- 130: a processor system
- 140: storage
- 150: communication interface

- 200: a risk stratification device
- 210: a sequencing data source
- 211: sequencing data
- 220: a first risk score unit
- 221: a sequencing data database
- 230: a folding unit
- 231: folding data
- 232: a folding database
- 233: a folding computer
- 234: a structural features vector
- 240: a risk score unit
- 241: a risk score
- 242: a second risk score database
- 243: a risk score computer
- 244: a further risk score
- 245: further risk data
- 246: structural feature identifier
- 250: a device
- 251: a protein identifier
- 252: a protein specific risk score unit
- 260: a risk score compositor
- 261: a first risk score unit
- 262: a second risk score unit
- 263: a combined risk score

- 1000, 1001: a computer readable medium
- 1010: a writable part
- 1020: a computer program
- 1110: integrated circuit(s)
- 1120: a processing unit
- 1122: a memory
- 1124: a dedicated integrated circuit
- 1126: a communication element
- 1130: an interconnect
- 1140: a processor system

### DESCRIPTION OF EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them. Further, the subject matter that is presently disclosed is not limited to the embodiments only, but also includes every other combination of features described herein or recited in mutually different dependent claims.

Embodiments may be applied to diseases in which incorrect folding plays a role, e.g., diseases with a genetic basis in the subject, in particular cancer, e.g., a particular form of cancer. A disease with a genetic basis is a disease in which incorrect folding is part of the disease etiology. There is evidence of improper protein folding influencing the pathogenesis of several diseases, including malignancies. For example, the cancer may be caused by misfolding of the p-53 protein; for example, caused by mutations at the genetic sites R175, G245, R248, R249, R273 and R282. Some further non-exhaustive examples include:
Misfolded tumor suppressors are inactive and lead to cancer because of a loss-of-function phenotype, as in the case of tumor-suppressor proteins VHL and NF2.

The mutated proteins may adopt an aberrant conformation that is regulated differently than the wild-type protein, e.g., dominant-negative inactivation of the wild-type tumor suppressor, as in the case of p53 and WT1.

Variant behavior of ubiquitin-proteasome system & chaperones: HSP90, HSP70 due to change in cell compartment homoeostasis, e.g., hypoxia, may lead to downstream effects e.g., VEGF transcription promotion.

Misfolding of c-Abl and p-53 in various cancers, without direct mutation, but in mutation in upstream control points.

See also, 'Protein-misfolding diseases and chaperone-based therapeutic approaches' by Tapan K. Chaudhuri and Subhankar Paul. There are public databases, e.g., the Protein Data Bank, of experimentally determined protein tertiary and quaternary structure. Traditionally, determining the tertiary folding structure of a protein involved, e.g., X-ray crystallography and subsequent analysis, cryogenic electron microscopy, and so on. In silico prediction of protein structure from the primary structure has been computationally challenging. However, accurate folding prediction is currently feasible with realistic computation/time, e.g., using machine learning approaches, e.g., using the AlphaFold model. Note that programs such as AlphaFold do not provide a prediction of the effect of a particular protein's folding.

Embodiments described herein enable clinicians to benefit from protein structure prediction from genome or transcriptome data. This is beneficial in situations, e.g., when a mutation, especially a novel mutation, in the protein coding part of a gene results in a difference in the peptide sequence, but the effect of such a difference has not been characterized. A novel mutation, possibly in tumor suppressor genes, chaperones, and heat shock proteins, etc., may have a non-obvious impact. Embodiments help to estimate the possible effect of the mutation. Note that the mutation may be in exons, but also in non-exon regions.

In situations where there is only aberrant folding of a protooncogene/tumor suppressor gene product, no mutations may be observed in their coding genes. However, analysis with an embodiment may identify the upstream agents, such as chaperones and heat shock proteins, etc., that are responsible.

Figure 1 schematically shows an example of an embodiment of a device for risk stratification 110. For example, the device for risk stratification 110 of figure 1 may be used to assign a risk score to an individual patient based on sequencing data obtained for that patient.

Device for risk stratification 110 may comprise a processor system 130, a memory 140, and a communication interface 150. Device for risk stratification 110 may be configured to communicate with a data storage. Storage may be a local storage of system 110, e.g., a local hard drive or memory. Storage may be non-local storage, e.g., cloud storage. In the latter case, storage may be implemented as a storage interface to the non-local storage.

Device 110 may communicate with external storage, input devices, output devices, and/or one or more sensors over a computer network. The computer network may be an internet, an intranet, a LAN, a WLAN, etc. The computer network may be the Internet. The systems comprise a connection interface which is arranged to communicate within the system or outside of the system as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna. Communication interface 150 may be used, e.g., to obtain transcriptomic and/or genomic sequencing data for at least one biological sample of a subject, e.g., a patient.

Processor system 130 may be configured to obtain a risk score indicating a risk for a disease with a genetic basis in the subject. Various ways to do this are discussed herein; a particular way to obtain a risk score is to apply a machine-learning model to at least folding data corresponding to the sequencing data. The folding data indicates an expected 3d-structure of the protein after synthesis.

The execution of device 110 may be implemented in a processor system, e.g., one or more processor circuits, e.g., microprocessors, examples of which are shown herein. The figures show functional units that may be functional units of the processor system. For example, figures 2a-2c, 3, and 4 may be used as a blueprint of a possible functional organization of the processor system. The processor circuit(s) are not shown separate from the units in these figures. For example, the functional units shown in the figures may be wholly or partially implemented in computer instructions that are stored at device 110, e.g., in an electronic memory of device 110, and are executable by a microprocessor of device 110. In hybrid embodiments, functional units are implemented partially in hardware, e.g., as coprocessors, e.g., neural network coprocessors, and partially in software stored and executed on device 110. Parameters of the network and/or training data may be stored locally at device 110 or may be stored in cloud storage.

Typically, device 110 comprises a microprocessor which executes appropriate software stored at the system; for example, that software may have been downloaded and/or stored in a corresponding memory, e.g., a volatile memory such as RAM or a non-volatile memory such as Flash. Alternatively, the systems may, in whole or in part, be implemented in programmable logic, e.g., as field-programmable gate array (FPGA). The systems may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc. In particular, device 110 may comprise circuits for the evaluation of neural networks.

A processor circuit may be implemented in a distributed fashion, e.g., as multiple sub-processor circuits. A storage may be distributed over multiple distributed sub-storages. Part or all of the memory may be an electronic memory, magnetic memory, etc. For example, the storage may have volatile and a non-volatile part. Part of the storage may be read-only.

Figure 2a schematically shows an example of an embodiment of a device 200 for risk stratification. The device for risk stratification comprises at least one computer hardware processor to perform the risk stratification. Typically, the risk stratification is done by assigning a risk score to a subject, e.g., a patient. Patient management can be informed by such a risk score; for example, patients with higher risk scores may be checked on more frequently, or with priority.

For example, if a risk score is obtained for a particular subject that is particularly high, the clinician may be informed and/or the subject may be invited for follow-up research.

Risk stratification enables healthcare providers to determine the appropriate level of care and services for different patient subgroups. For example, risk stratification may comprise assigning a risk score to patients and then using that score to guide care and improve overall health outcomes. Risk stratification allows treating patients both as individuals and as members of a larger community or population. For example, a patient's risk score, or a category in which the patient may be grouped based on his/her risk score, allows planning, developing, and implementing an individualized care plan. One of the common methods of segmenting patients is the level of risk, e.g., into the levels: high, medium, and low risk.

Risk stratification enables the personalization of models of care to meet the needs of patients in each subgroup. This can improve clinical outcomes, and/or reduce costs. For example, high-intensity resources may be reserved for high-risk patients. Risk-based models of care allows appropriately matching needs with resources. Risk stratification may separate patients into separate groups with similar complexity and care needs. Risk stratification may be more careful, if the model indicates low confidence values for a risk score.

Figure 2a shows sequencing data 211. Sequencing data 211 is transcriptomic and/or genomic sequencing data for at least one biological sample of a subject. In the subject, the sequencing data codes for a protein synthesizable in the subject. For example, the sequencing data may represent DNA data, or mRNA data. In the subject the DNA data, or mRNA data would cause the synthetization of protein. Obtaining and identifying proteins in a subject, although possible in principle, is much harder than obtaining the precursing sequencing data. Embodiments can be applied both the DNA and RNA data. Using RNA data or transcriptomic sequencing data has the advantage that better information on co-occurring proteins can be obtained, since analysis of from an RNA sample from a tissue automatically filers out nontranscribed/silent regions of the genome.

Figure 2a shows a sequencing data source 210 from which the sequencing data 211 is obtained. The sequencing data source 210 could for example, be a genomic or transcriptomic sequencing device. The sequencing data source 210 could, for example, be a patient database storing sequencing data. As indicated by the dotted line, the sequencing data source 210 may be part of risk stratification device 200, but this is not necessary, or even typical.

Sequencing data 211 may correspond to a single protein. A single sequence of sequencing data is a typical use case. For example, the sequencing data may correspond to a mutated RNA or DNA sequence resulting in modified transcriptomic and/or genomic sequencing data. The mutation may cause an incorrect folding, which in turn may lead to disease, in particular to cancer.

Figure 2a shows two sequences 211. A practical embodiment may well accept a single sequence, e.g., a sequence, or part thereof, coding for the relevant protein. For example, the protein may be known to be important for a genetic disease, e.g., cancer, and/or the sequence may be known to contain a mutation, especially a novel mutation.

In an embodiment, the sequence data or folding data is used to identify the protein. Based on the protein a decision on further processing can be made by the risk stratifying device. For example, if the gene/protein is not a, possibly mutated, version of a gene/protein related to the disease, e.g., to cancer, its processing can be skipped. For example, if the gene or protein is recognized as a, possibly mutated, particular gene or protein version then processing may be done by a machine learning algorithm that is specially configured for that protein.

Sequencing data 211 may correspond to multiple, typically related, proteins. Often multiple proteins form a complex of mutually influencing proteins. An incorrect folding in a protein may increase disease risk moderately in some subjects, but will increase risk much more depending on the particular co-occurring proteins; even if said set of co-occurring proteins are folded correctly.

An embodiment may also accept multiple sequences, e.g., as shown. For example, the sequences, or part thereof, may code for co-occurring proteins. For example, the co-occurring proteins may occur in the same biological structure, tissue, cell, plasma, or the like. For example, co-occurring proteins may have a functional interplay. For example, co-occurring proteins may include proteins that are modified together, e.g., in post-translational modifications. One or more or all of the sequences may show mutations, e.g., novel mutations, though this is not necessary. For example, the particular set of proteins, co-occurring with a mutated one may increase or decrease the risk score.

For example, the sequence data 211 may be obtained by receiving or retrieving the sequencing data. The obtaining could comprise a sequencing method. For example, sequencing data source 210 may be comprised in risk stratification device 200; although that is optional and not typical.

The sequencing data may originally be obtained from a blood sample, salvia sample or other biological sample. As a few illustrating examples, the obtained sequencing data may correspond to
- a tumor suppressor, for example, tumor-suppressor protein VHL, NF2, p53 and/or WT1,
- an ubiquitin-proteasome system protein and/or chaperone, in particular HSP90, HSP70, and/or
- the ABL1 or c-Abl protein.

See also the article cited in the background.

Risk stratification device 200 comprises an optional first risk score unit 220. Risk stratification device 200 comprises a sequencing data database 221. First risk score unit 220 allows integration of folding based method with sequence data based method to establish a risk score. For example, first risk score unit 220 may determine from a genomic database 221 if the sequencing data 211 is known. For example, the sequencing data 211 may represent a known allele, a known mutation, or the like. If sufficient data is available concerning sequencing data 211 from a database, like sequencing data database 221, then first risk score unit 220 may compute a risk score directly from the sequential data. For example, the risk score may indicate how malignant, etc., an associated cancer is likely to be.

For a well-documented gene mutation, the risk may be calculated statistically. If sufficient data is available the folding may be skipped altogether, as a risk score is then available. Nevertheless, even in this case obtaining folding data is advantageous. For example, obtaining a folding based risk score may confirm or dispute the database based score.

Furthermore, protein structure, whether determined by classical methods such as wet-lab techniques and X-ray crystallography or by in-silico methods, can be used as ground truth/training data for the models, e.g., together with statistically obtained scores to these proteins, or protein ensembles or aggregates.

First risk score unit 220 may be omitted. For example, sequencing data 211 could be directly transmitted to folding unit 230, described herein.

Risk stratification device 200 comprises a folding unit 230. Folding unit 230 is configured to obtain folding data 231 corresponding to the sequencing data, to at least one of the sequencing data in case there are multiple sequencing data.

For example, in an embodiment sequencing data 211 comprises a single sequence X1 corresponding to a protein x1. In this case, folding unit 230 may obtain folding data indicating an expected folding of protein x1.

For example, in an embodiment sequencing data 211 comprises multiple sequences, e.g., X1, X2, ..., corresponding to protein x1, and co-occurring proteins, e.g., chaperones, x2, .... In this case, folding unit 230 may obtain folding data indicating an expected folding of protein x1. Folding unit 230 may also obtain folding data indicating an expected folding of one or more of the other proteins x2, .... Obtaining folding data for multiple or all of the multiple sequences is advantageous, but not necessary; for example, in an embodiment, folding unit 230 is configured not to obtain folding data for at least some, or all of, the co-occurring proteins x2, .... Note that even the unfolded proteins may be used as a further input for the risk score computation; further discussed herein.

Folding data 231 indicates an expected 3d-structure of the protein after synthesis. For example, a sequence data X may correspond to transcriptomic and/or genomic sequencing data. The corresponding transcriptomic and/or genomic molecule, e.g., an RNA molecule, e.g., an mRNA molecule, or DNA molecule is expected to naturally synthesize in vivo to a corresponding protein. The folding data indicates the expected 3d-structure of the synthesized corresponding protein.

For example, the folding data 231 may comprise a list of coordinates indicating the position of atoms in the folded structure, e.g., their 3D location in space, e.g., atomic coordinates. The folding data may comprise additional information, e.g., about the structure, sequence, and/or how the data was obtained.

For example, an atom in the folding data may be identified by one or more of a sequential number, a specific atom name, a name and number of a residue the atom belongs to, an identifier specifying a chain the atom belongs to. The identified atom may have coordinates, e.g., x, y, and z coordinates, polar coordinates, or the like. Further information may comprise an occupancy and temperature factor.

Conventional data formats may be used to implement folding data. For example, the folding data 231 may be in the PDB file format, mmCIF format, PDBx/mmCIF format, XML format, and so on. A proprietary format may be used instead of a conventional format.

Folding unit 230 has several options to obtain the folding data. One option is to retrieve the folding data from a folding database 232. For example, folding database 232 may be obtained by collecting folding information for various sources. The folding data in the database may be obtained from experiment, e.g., crystallographic, etc. folding data in the database may be obtained from in silico folding. For example, folding database 232 may be comprised in device 200, e.g., in folding unit 230.

For example, protein structures may be collected as PDB formatted files and obtainable from such databases, however the databases do not contain all protein structures possible in a cancer patient.

For example, public databases, e.g., the Protein Data Bank, may be used that comprises experimentally determined protein tertiary and quaternary structure. Determining the tertiary folding structure of a protein may involve, e.g., X-ray crystallography and subsequent analysis, cryogenic electron microscopy, and so on.

Another option for folding unit 230 to obtain folding data is to use a folding computer 233, e.g., a folding function, folding algorithm, or the like. Folding computer 233 may comprise software configured to compute an expected folding given sequencing data. For example, folding computer 233 may be configured to obtain folding data 231 by applying an in-silico protein folding prediction algorithm to the sequencing data. For example, folding data 231 may comprise one or more machine learning algorithms trained to predict folding data from sequencing data. For example, the one or more machine learning algorithms may comprise neural networks, e.g., deep neural networks.

In silico prediction of protein structure from the primary structure used to be computationally challenging. However, accurate folding prediction is currently feasible with realistic computation and time, e.g., using machine learning approaches. A particular example is the AlphaFold model. Note that a conventional folding computer, like AlphaFold, enables folding prediction from available genome or transcriptome information, but does not provide functional effect prediction. More information on the AlphaFold model may be obtained from the paper "Highly accurate protein structure prediction with AlphaFold", by J. Jumper et al.

Risk stratification device 200 comprises a risk score unit 240. Risk score unit 240 is configured to obtain a risk score 241 from the folding data indicating a risk for a disease with a genetic basis in the subject. Risk score unit 240 is configured to receive as input folding data 231 for at least one sequencing data 211.

The risk score may indicate an expected likelihood for the disease with a genetic basis of an occurrence, severity, recurrency, and/or disease-specific death in the subject. Protein misfolding is believed to be the primary cause of Alzheimer's disease, Parkinson's disease, Huntington's disease, Creutzfeldt-Jakob disease, cystic fibrosis, Gaucher's disease and many other degenerative and neurodegenerative disorders.

An embodiment may be applied to any of these diseases; however, a particularly advantageous disease for applying an embodiment is cancer. The disease with a genetic basis may be cancer. The risk score may indicate a cancer risk score in the subject. For example, the risk score may be a cancer risk score and indicate an expected likelihood of a cancer occurrence, cancer malignancy, cancer recurrence, and/or cancer-specific death in the subject.

The risk score that is computed may be forwarded to a further device 250. For example, a report may be generated. Forwarding may be subject to exceeding a threshold. Device 250 may be a display, an e-mail server, or the like. Further device 250 may be part of device 200, but is typically not.

Risk stratification device 200, e.g., risk score unit 240, comprises a risk score computer 243. Risk score computer 243 is configured to receive as input at least one folding data 231, e.g., corresponding to at least one sequencing data 211, and to produce as output a risk score 241, e.g., a genetic disease risk score, such as a cancer risk score. In the shown embodiment, the folding computer 233 is shown separate from the risk score computer 243; the folding computer 233 is shown separate could also be incorporated in the risk score computer 243.

Having a risk score computer 243 is advantageous since the sequence 211 may be unique, or at least novel to device 200. This means that no statistical data is available for the particular sequence. Statistical methods are likely to fail or perform poorly on sequencing data that are encountered for the first time. However, having folding data, that is, having an indication of the folded protein, a much better estimate can be given, even for a sequence encountered for the first time.

For example, risk score computer 243 may compare the folded protein with folding data for one or more known alleles of the protein for which risk scores are known. One or more similarity scores between the folded protein represented by folding data 231 and the allele folding data may be obtained. The similarity score or similarity scores may be combined with known risk scores for the alleles. Accordingly, if folding data 231 is similar to a known folding with high risk, then a correspondingly high risk score 241 may be computed. For example, a similarity function SIM may take as input folding data x1 and y1, wherein x1 is folding data 231 and y1 is folding data for a known allele. For example, a=SIM(x1, y1) may be computed by risk score computer 243. The similarity score a indicates a 3D similarity in the folded states of the proteins. Note that a small change in the sequencing data X1 may give rise to large change in the folding data x1, and vice versa. An algorithm operating on sequencing data X1 and Y1 may be misled, where an algorithm operating on folding data is not. The similarity score may be translated to a risk score, e.g., b =TRANS (a, ya), wherein ya is the known risk score for y1 and b is the risk score for x1. For example, TRANS may multiply a^{∗}ya; other functions are possible.

Better risk score estimates can be obtained by using a machine learning algorithm. For example, in an embodiment, risk score computer 243 is configured to apply a machine-learning model to at least the folding data 231 to generate a risk score 241.

For example, risk score unit 240 may be trained on multiple training items. A training item may comprise folding data and a risk score. The folding data in a training item may be obtained from in-silico methods, like folding computer 233 or from experimental data, or both. The risk data may be obtained from a statistical method. For example, for a known mutation which is a known cancer risk, a risk score can be established from a database of outcomes of people that do and do not have the known mutation. Furthermore, folding data for the known mutation may be obtained, e.g., from a folding computer 233. Together the folding data and risk score form a training item.

Training data, e.g., a multiple of training items, may be stored in an optional second risk score database 242. Interestingly, as device 200 is used, folding data and outcome data may be accumulated, and stored, e.g., in second risk score database 242. The machine learning model 243 may be updated, e.g., refined on the accumulated data, or may be fully retrained on the training data and the accumulated new data.

In an embodiment, the risk score computer 243 comprises a neural network, in particular a deep neural network. Risk score computer 243 may comprise a convolution neural network, e.g., comprising multiple convolutional layers. For example, the convolutional layers may learn to recognize structural features in the folding data and associate these with higher or lower risk scores. The neural network may use multiple 2D convolutional layers. Interestingly, the neural network may comprise one or more 3D convolutional layers.

In an embodiment, the risk score computer 243 comprises another machine learning model than a neural network (or in addition thereto). For example, risk score computer 243 may comprise a decision tree, random decision forests, gradient boosting random forest or the like. For example, risk score computer 243 may comprise an SVM model.

In an embodiment, a machine learning model receives essentially the folding data as an input, but not necessarily much other structural information concerning the corresponding protein. In another embodiment however, structural features are identified for the protein from the folding data and provided to the model.

For example, the structural features may comprise: α-helices, β-sheets, β-strands, parallel β-strands, anti-parallel β-strands. Furthermore, information such as the number of the structural features found, whether they occur on the periphery, or in a backbone, and so on, may be collected. For example, given a folding data x, a structural information vector xx = (xx1, xx2, ... ) may be generated. The elements in the vector indicating presence, and/or number, and/or location of structural features of the protein represented by the folding data. The structural information vector may be provided as an additional input to the machine learning risk score computer 243. Providing a structural information vector to a machine learning algorithms is advantageous as these are computationally involved features to compute for a machine learning algorithms, but are easier to compute with a structural analysis program, possibly a conventional structural analysis program. For example, such structural features may conform to a structure classification, e.g., SCOP, CATH, etc.

Computing structural information, e.g., a structural information vector, may be used both for neural network and non-neural network type machine learning models; computing structure features for the model is especially beneficial for non-deep neural networks.

If the risk score computer 243 comprises a deep neural network, the structural information vector is less needed, and can even be disadvantageous, as the deep neural network is capable of identifying the structural features that correlate to risk score. If the risk score computer 243 has fewer computation resources, e.g., a decision tree, SVM, or the like, the structural information vector is more advantageous, as the machine learning algorithm is less capable of identifying the structural features that correlate to risk score by itself.

In an embodiment, the machine learning algorithm also determines a confidence value for a risk score. For example, a confidence value may represent the amount of relevant training data-e.g., how often similar folding data is represented in the training data. Alternatively, a confidence value may be computed using conventional algorithms, for example, a confidence value may be computed using dropout in a neural network.

Interestingly, a protein specific risk factor, e.g., a risk factor determined only from folding data for one protein, can be combined with other risk factors, e.g., a factor computed from the co-occurring proteins. For example, severity, in particular severity of malignancy, e.g., likelihood to spread in the subject, may be affected by co-occurring proteins.

If a risk factor, e.g., risk factor 241, is found that exceeds a threshold, then a warning signal may be generated; for example, a report may be generated. For example, the report may comprise a reference to the sequencing data, and/or the folding data that caused the warning signal. This allows a medical professional to refer back to the original data and verify if the warning is justified. Whether the assigned risk score is clinically significant may further be judged through the natural history of the disease/prognosis of the patient and the score adjusted accordingly.

As an example, when a novel reading frame, is observed, the possible resulting protein structure may be imputed, e.g., using a deep learning-based protein structure prediction algorithm, scored using the risk score computer 243, and added to the framework database. Further information, e.g., outcome data, may be stored in association with the resulting protein structure. Such data can be used for training a risk factor prediction model.

Risk score computer 243 may comprise a machine-learning model, which is applied at least to the folding data. Known examples of proteins whose coding sequence in the genome, structures, and severity of associated disease and/or pathway interactions are characterized may be obtained. A machine learning model may be trained so that a novel protein observed in future can be judged to be similar in structure and function to previously observed proteins

A risk factor may be computed solely from a single folding data 231, e.g., corresponding to a single suspect sequencing data 211. Interestingly, better predictions are obtained by supplementing folding data 231 with additional data.

For example, risk score unit 240 may obtain transcriptomic and/or genomic sequencing data for co-occurring proteins in the subject. Folding data may, or may not, by obtained for the further sequencing data. The co-occurring proteins may be represented with an identifier rather than with sequencing data. Co-occurring proteins may or may not be mutated.

Additional data may be received as an additional input to the risk score computer 243. For example, a machine learning model may be trained to receive a variety of data and compute a risk score therefrom.

For example, in an embodiment, the risk score computer 243 comprises a Long Short-Term Memory Recurrent Neural Networks (LSTM). Long Short-Term Memory (LSTM) networks are a type of recurrent neural network capable of learning to predict a value from a sequence of data. For example, a disease risk prediction may be presented with folding data, but also with other relevant data. This may be genomic and/or transcriptomic but may also be other data from a patient's medical record.

For example, a series of records *xᵢ* may be presented, at least one of which comprise folding data. A recurrent neural network, e.g., processes each of the records in turn, updating a hidden state after each processed record. An output may be derived from the final hidden state. This architecture can be adapted to accept a range of different types of risk data, including folding data but also test results, other risk factors and the like.

Risk score unit 240 may be configured to receive risk data 245 from other sources; A further risk score may be derived from the further risk data 245. For example, a further risk score 244 may be received. A further risk score may be combined with a risk score computed from folding data. A further risk score and/or other further risk data may be received as an additional input in the risk score computer 243

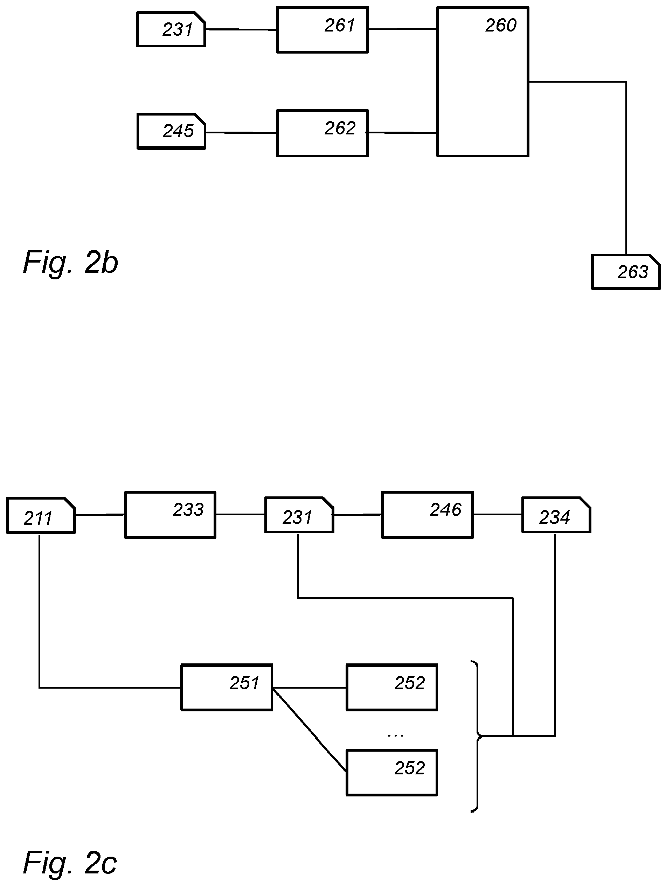

One way to take into account multiple types of data is represented in figure 2b. Figure 2b schematically shows an example of an embodiment of a device for risk stratification similar to the one shown in figure 2a.

Shown in figure 2b are multiple risk score units. Shown are a first risk score unit 261 and a second risk score unit 262. For example, first risk score unit 261 may receive folding data 231, while second risk score unit 262 may receive other data, e.g., test results. The multiple risk score units each compute a risk score based on the information available to them. This architecture provides a flexible alternative to a large model that is configured to receive all types of risk data.

Shown in figure 2b is a risk score compositor 260 of the risk stratification device. For example, risk score compositor 260 may be integrated in risk score unit 240. Risk score compositor 260 is configured to obtain multiple risk scores indicating a disease risk in the subject and to compute a combined risk score 263 from the multiple risk scores. Risk scores may be combined using a Bayesian formula, or using some other statistical or arithmetic formula.

For example, risk score compositor 260 may receive a protein specific risk score, e.g., computed from one folding data obtained from one sequencing data. For example, risk score compositor 260 may receive a co-occurring protein specific risk score, e.g., computed from co-occurring proteins that co-occur with the protein associated with the protein specific risk score.

Risk factors that are received by the risk score compositor 260 may also be data that is not directly genomic/ transcriptomic, e.g., clinical data, a clinical classification, e.g., a National Comprehensive Cancer Network (NCCN) classification, Cancer of the Prostate Risk Assessment, Gleason score, PSA level, and so on.

For example, risk scores may be combined with various types of functions. For example, the function may compute an average, possibly weighted. For example, the function may compute a maximum. For example, the function may compute a different type of combining function, e.g., a harmonic average. The combining function may be hand-tuned, or machine-learned.

Figure 2c schematically shows an example of an embodiment of a device for risk stratification. Figure 2c illustrates yet a further advantageous architecture. Shown in figure 2c is sequencing data 211. Shown is one such data 211, but there may be more. A folding computer 233 configured to compute corresponding folding data 231 indicating the 3D structure of the protein encoded by sequencing data 211 after synthesis. A structural feature identifier 246 is configured to identify in the folding data 231 any of a list of structural features, e.g., alpha helices, beta sheets, and so on, as indicated herein. The identified features may be represented in a suitable format, such as a structural features vector 234. A protein identifier 251 is configured to identify the protein encoded by sequencing data 211. Protein identifier 251 may compare the sequencing data with known sequences for known proteins, and identify a known protein that is close. For example, protein identifier 251 may identify the p53 gene, even though, it may be mutated. Protein identifier 251 may also (or instead) receive the folding data 231 and/or the structural features 234 as input. Based on the identified sequence an appropriately trained machine learning model is selected. Shown are two such models: protein specific risk score unit 251 and protein specific risk score unit 252.

A protein specific risk score unit may receive the structural feature data 234 and derive therefrom a risk score. A protein specific risk score unit may also (or instead) receive the folding data 231.

For example, protein identifier 251 may recognize p53 in the sequencing data 211, and apply a p52 specific model on structural feature vector 234; obtaining a risk factor. This is an advantageous model as it allows specialized model to be trained on situations for which good data is available.

Further optional improvements include one or more of the following:
For example, protein identifier 251 may recognize sequence data 211 as not relevant for cancer, and cause the sequence data 211 to be reported as not important.

For example, protein identifier 251 may recognize sequence data 211 as a co-occurring protein, and cause the sequence data 211 to be scored as a further risk factor.

For example, protein identifier 251 may recognize sequence data 211 as not potentially relevant for cancer. However, a model for the protein may not be available. The situation may be reported, and folding data may be stored. When sufficient data is available a new model may be trained.

Below several further optional refinements, details, and embodiments are illustrated.

A possible high-level flow of a risk stratifying method is described:

### Framework construction

Selecting examples of proteins whose primary, secondary, and tertiary structures are documented and functional differences between wild type protein and the variant observed in malignancy difference are characterized.

Obtain the protein structures, e.g., in PDB format

### Model training:

(A) AI: Train an AI model (e.g., implicit feature extraction) with the known structures, with the severity of malignancy to which the variant is associated used as a label/score. The model may be configured to assign a score to a novel structure by identifying similar structures. The similarity can be structural similarity or similarity of active domains, e.g., leading to function. In addition to individual protein structures, significant correlations (co-occurrences in the same biopsy sample) may be considered as well.
   Alternatively model training may use
   (B) Traditional machine learning:
   (i) Extract features from the secondary/tertiary structures and dimensionality reduction, e.g., PCA, if needed.
   (ii) For a given malignancy/disease, all observed structures are assigned an initial score according to the severity of the disease.
   (iii) The structures are clustered using the extracted features and composite scores of the clusters are calculated based on the scores of the structures that constitute a cluster, e.g., mean of the scores.

Each of these known proteins may be assigned a score using multiple parameters, e.g.,

Severity of associate malignancy, if any, contributes to the individual protein's score

Scores of the co-occurring proteins contribute to another component of the score Final score is calculated as a weighted average of these two.

A database is constructed to record the DNA/RNA sequence, primary, secondary, tertiary, and quaternary structures, associated malignancy and severity, co-occurring proteins (and other pathway information) for novel structures to be observed in future.

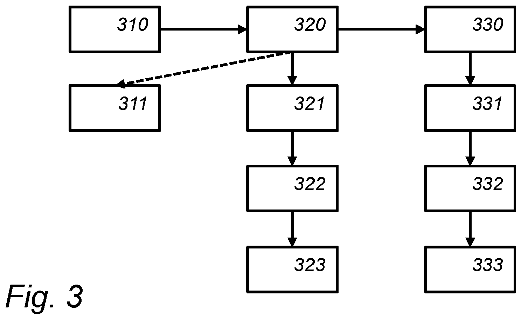

Figure 3 schematically shows an example of an embodiment of the model construction. Shown in figure 3
310: selecting proteins with a characterized structure and function.
320: obtaining folding data for the selected protein.

Starting from 320 two paths are followed. To obtain training data one or more of the following may be implemented:
330: assigning a risk score according to severity of associated malignancy. This may be based on existing evidence.
331: assigning a score to co-occurring proteins
332: estimating a final score from individual aggregate components
333: recording sequence, folding structure, and scores in a database

To train a risk score computer one or more of the following may be implemented:
Option A
   311: Train an AI model directly with folding data, e.g., PDB files, to estimate similarity of a novel structure to existing ones.
Option B
   321: Feature extraction and selection
   322: Clustering
   323: Model to estimate similarity of a novel structure to existing ones

Once the model is generated, it may be used to process a candidate genome/transcriptome. For example, this may comprise the following parts:
Genome/transcriptome sequencing may be performed using appropriate samples as appropriate (e.g., tissue biopsy sample for transcriptome analysis and biopsy or blood sample for genome analysis) with established procedures.

Reading frames are extracted from the genome/transcriptome

If the RF was previously observed:
The RF's individual score is considered

Any difference in the associated (co-occurring) proteins is analyzed. If no difference is observed, the previous score is considered.

If there is any difference in the co-occurring proteins, the aggregate's score is estimated based on most similar aggregates. The score can be updated later based on the patient's prognosis. If the RF is novel:
The corresponding protein secondary, tertiary, and quaternary (if applicable) structures are determined using a deep learning-based protein folding algorithm e.g., AlphaFold.

Depending on the framework used, either the structure can directly be analyzed with the model mentioned in option (A) or features extracted as in option (B).

The RF is scored by the framework by:
Either directly (A) or through its distances from the clusters (B)

From co-occurring proteins.

Initially the score would be given a low confidence value.

It may be noted that following this workflow would result in performing the protein structure prediction a rare occurrence (only when required), but the score update would be more frequent. As any estimated structures and scores would be memorized, redundant computation would be avoided.

Optionally score the whole genome/transcriptome for malignancy likelihood.

The hospital/clinician can be alerted if a novel ORF produces a high score indicating the possibility of severe disease. If the high score is validated based on the prognosis of the patient or multiple patients, the score would have a high associated confidence.

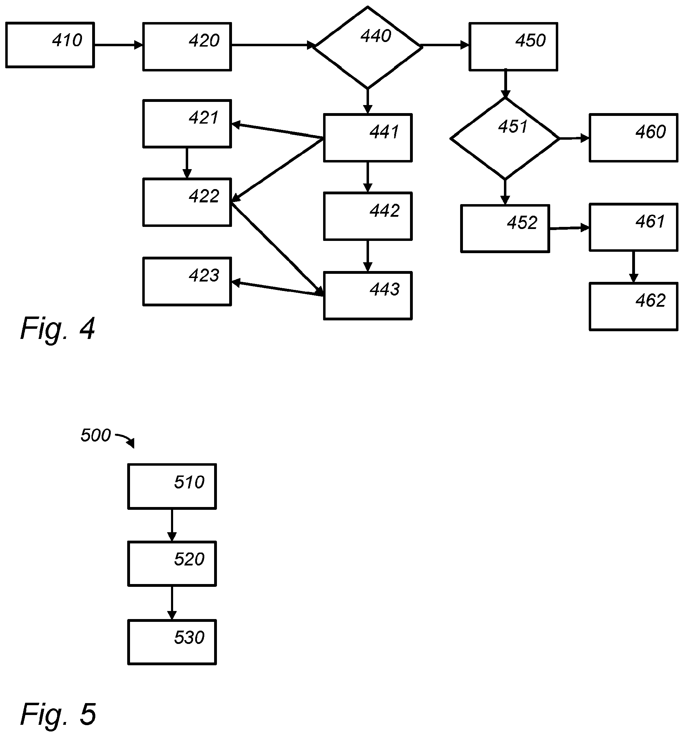

Figure 4 schematically shows an example of an embodiment of the processing of a novel structure. Shown in figure 4
410: Genome/Transcriptome sequencing
420: Extracting reading frames
440: Determining if sequence is novel
450: (path taken for No answer at 440) Lookup predicted protein structure, co-occurrence, and score in database
451: Determining if co-occurring proteins are different
460: (path taken for No answer at 451) Use score for risk stratification. Update confidence and evidence
452: (path taken for Yes answer at 451) Re-estimate score component from the co-occurring proteins
461: Monitor prognosis
462: Update evidence/ confidence value
441: (path taken for Yes answer at 440) Determine primary and secondary structure. Predict tertiary structure.
421: extract features from tertiary structures
422: Score individual component using developed machine learning model
442: Score aggregate component using co-occurring protein scores
443: Generate final score, with low confident score
423: Use score in risk stratification.

Figure 5 schematically shows an example of an embodiment of method 500 of risk stratification. Method uses at least one computer hardware processor. Method 500 comprises
- obtaining (510) transcriptomic and/or genomic sequencing data for at least one biological sample of a subject, the sequencing data coding for a protein synthesizable in the subj ect,
- obtaining (520) folding data corresponding to the sequencing data, the folding data indicating an expected 3d-structure of the protein after synthesis,
- applying (530) a machine-learning model to at least the folding data obtaining a first risk score indicating a risk for a disease with a genetic basis in the subject.

Many different ways of executing the methods described herein are possible, as will be apparent to a person skilled in the art. For example, the order of the steps can be performed in the shown order, but the order of the steps can be varied or some steps may be executed in parallel. Moreover, in between steps other method steps may be inserted. The inserted steps may represent refinements of the method such as described herein, or may be unrelated to the method. For example, some steps may be executed, at least partially, in parallel. Moreover, a given step may not have finished completely before a next step is started.

Embodiments of the method may be executed using software, which comprises instructions for causing a processor system to perform the methods described herein, e.g., shown with respect to figures 3, 4, and 5. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

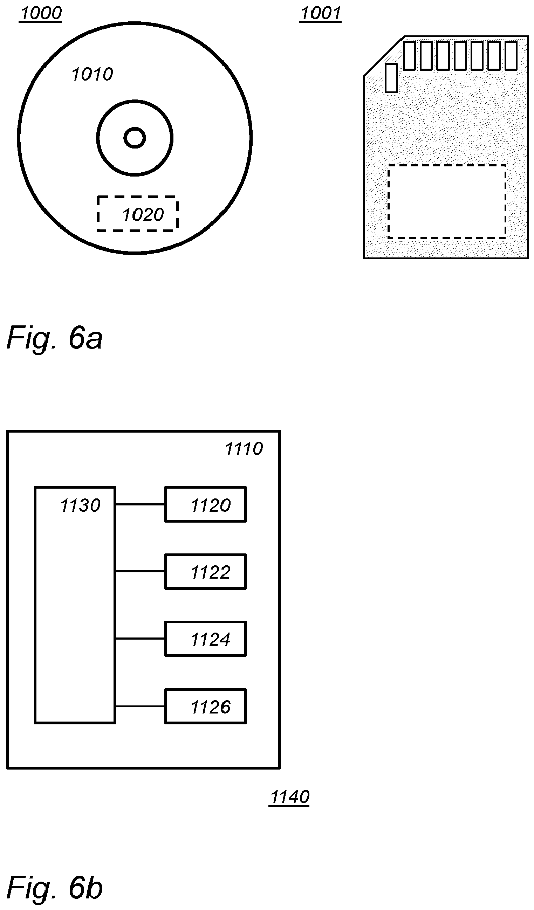

Figure 6a shows a computer readable medium 1000 having a writable part 1010, and a computer readable medium 1001 also having a writable part. Computer readable medium 1000 is shown in the form of an optically readable medium. Computer readable medium 1001 is shown in the form of an electronic memory, in this case a memory card. Computer readable medium 1000 and 1001 may store data 1020 wherein the data may indicate instructions, which when executed by a processor system, cause a processor system to perform an embodiment of a method of risk stratification, according to an embodiment. The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said method of risk stratification.

Figure 6b shows in a schematic representation of a processor system 1140 according to an embodiment of a risk stratification device. The processor system comprises one or more integrated circuits 1110. The architecture of the one or more integrated circuits 1110 is schematically shown in Figure 6b. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively.

For example, in an embodiment, processor system 1140, e.g., the risk stratification device may comprise a processor circuit and a memory circuit, the processor being arranged to execute software stored in the memory circuit. For example, the processor circuit may be an Intel Core i7 processor, ARM Cortex-R8, etc. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of' when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

## Claims

1. A method (500) of risk stratification comprising, using at least one computer hardware processor to perform:
- obtaining (510) transcriptomic and/or genomic sequencing data for at least one biological sample of a subject, the sequencing data coding for a protein synthesizable in the subj ect,
- obtaining (520) folding data corresponding to the sequencing data, the folding data indicating an expected 3d-structure of the protein after synthesis,
- applying (530) a machine-learning model to at least the folding data obtaining a first risk score indicating a risk for a disease with a genetic basis in the subject.

2. A method as in Claim 1, wherein obtaining folding data comprises applying an in-silico protein folding prediction algorithm to the sequencing data.

3. A method as in any one of the preceding claims, wherein information indicating disease risk is stored in a database associated at least with the sequencing data.

4. A method as in any one of the preceding claims, comprising
- determining from a genomic database if the sequencing data is known, and
- if so, obtaining the first risk score from the genomic database,
- if not, obtaining the folding data, applying the machine-learning model, and obtaining the first risk score therefrom.

5. A method as in any one of the preceding claims, comprising generating a warning signal if the risk score exceeds a threshold.

6. A method as in any one of the preceding claims, comprising
- obtaining observed disease risk in a subject associated with folding data and training and/or retraining the machine-learning model to generate a risk score indicating the disease risk from at least folding data.

7. A method as in any one of the preceding claims, comprising
- obtaining transcriptomic and/or genomic sequencing data for co-occurring proteins in the subject, and obtaining a second risk score indicating a disease risk in the subject therefrom,
- computing a combined risk score from the first risk score and the second risk score.

8. A method as in any one of the preceding claims, comprising
- identifying structural features of the protein from the folding data, the machine-learning model receiving at least the identified structural features as input.

9. A method as in any one of the preceding claims, comprising determining a confidence value for a risk score.

10. A method as in any one of the preceding claims, wherein the disease risk is a cancer risk in the subject and indicates an expected likelihood of a cancer occurrence, cancer malignancy, cancer recurrence, and/or cancer-specific death in the subject.

11. A method as in any one of the preceding claims, wherein the obtained sequencing data corresponds to
- a tumor suppressor, for example, tumor-suppressor protein VHL, NF2, p53 and/or WT1,
- an ubiquitin-proteasome system protein and/or chaperone, in particular HSP90, HSP70, and/or
- the ABL1 or c-Abl protein.

12. A device for risk stratification comprising,
- a communication interface configured to obtain sequencing data
- at least one computer hardware processor configured to perform
- obtaining transcriptomic and/or genomic sequencing data for at least one biological sample of a subject, the sequencing data coding for a protein synthesizable in the subj ect,
- obtaining folding data corresponding to the sequencing data, the folding data indicating an expected 3d-structure of the protein after synthesis, and
- applying a machine-learning model to at least the folding data obtaining a first risk score indicating a risk for a disease with a genetic basis in the subject.

13. Transient or non-transient computer readable medium comprising computer instructions configured to perform any one of the methods of claims 1-11 when the computer instructions are executed on a computer.
